(19) Europäisches Patentamt
European Patent Office
Office européen des brevets

(11) EP 3 061 750 B1

(12) **EUROPEAN PATENT SPECIFICATION**

(45) Date of publication and mention of the grant of the patent:
**28.03.2018 Bulletin 2018/13**

(21) Application number: **13896120.6**

(22) Date of filing: **12.12.2013**

(51) Int Cl.:
***C07D 231/12*** *(2006.01)* ***A01N 43/56*** *(2006.01)*
***A01P 3/00*** *(2006.01)*

(86) International application number:
**PCT/CN2013/089220**

(87) International publication number:
**WO 2015/058444 (30.04.2015 Gazette 2015/17)**

(54) **PYRAZOLE AMIDE COMPOUNDS CONTAINING A DIPHENYL ETHER GROUP, AND APPLICATION THEREOF, AND PESTICIDE COMPOSITION**

PYRAZOLAMIDVERBINDUNGEN MIT EINER DIPHENYLETHERGRUPPE UND VERWENDUNG DAVON SOWIE PESTIZIDZUSAMMENSETZUNG

COMPOSÉS AMIDE DE PYRAZOLE CONTENANT UN GROUPE OXYDE DE DIPHÉNYLE ET LEUR APPLICATION ET COMPOSITION DE PESTICIDE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **23.10.2013 CN 201310502473**

(43) Date of publication of application:
**31.08.2016 Bulletin 2016/35**

(73) Proprietors:
- **Central China Normal University**
  **Wuhan, Hubei 430079 (CN)**
- **Beijing Yoloo Bio-technology Corp., Ltd**
  **Beijing 101105 (CN)**

(72) Inventors:
- **YANG, Guangfu**
  **Wuhan**
  **Hubei 430079 (CN)**
- **XIONG, Li**
  **Wuhan**
  **Hubei 430079 (CN)**
- **CHEN, Qiong**
  **Wuhan**
  **Hubei 430079 (CN)**

(74) Representative: **Geling, Andrea**
**ZSP Patentanwälte PartG mbB**
**Hansastraße 32**
**80686 München (DE)**

(56) References cited:
**WO-A1-2012/065947**
**CN-A- 1 226 244**
**CN-A- 101 056 858**
**DE-A1- 19 629 828**
**JP-A- 2004 189 738**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 061 750 B1

## Description

### Field of the invention

The invention relates to a pyrazole amide compound containing a diphenyl ether group, and specifically to application thereof in the prevention and treatment of rice sheath blight and cucumber powdery mildew, and pesticide composition containing the compound.

### Background of the invention

Rice is one of the most important food crops in our country, the planting area for rice accounts for about 30% of the area for grain crops, the yield of rice is nearly half of the country's total grain output. Rice sheath blight disease, which stands first among three major rice diseases, is the main obstacle in high and stable rice production. In recent years, with the popularization of the hybrid rice varieties with high yield or superhigh yield and increasing amount of nitrogen fertilizer for rice, the occurrence area of the rice sheath blight has expanded each year, and the extent of damage has increased year by year, and the serious loss in yield can be 50% or more. Cucumber is one of the important economic crops in China. It has the characteristics of high yield, good benefits and wildly planting area. Cucumber powdery mildew is one of the most common diseases in cucumber cultivation, it occurs all over the country, and breaks out all the year round, resulting in 10% -40% reduction in production of cucumbers. Currently, agents for prevention and treatment of these two diseases are mainly Jinggangmycin, difenoconazole, propiconazole and chlorothalonil, etc., but the single mechanism of action and long-term and extensive use of these pesticides have caused pathogenic bacteria to become serious drug resistance, resulting in poor prevention and control effect. Therefore, it is necessary to develop new bactericides which have good prevention and control effects on rice sheath blight and cucumber powdery mildew.

The existing research results show that part of the succinate dehydrogenase inhibitors (SDHIs) as a kind of bactericide have good prevention and treatment effects on rice sheath blight and powdery mildew, and the prevention and treatment effect of such bactericide is achieved by inhibiting the activity of pathogenic bacteria succinate dehydrogenase and causing death of the pathogenic bacteria, since it has a novel mechanism of action, such bactericide has no cross-resistance with most of bactericides available on the market. At the same time, it also has a very broad spectrum bactericidal activity, and can prevent and control a variety of diseases harmful to crops. Therefore, bactericide of succinate dehydrogenase inhibitors has become one of the most important class of bactericides, and it is the major research focus of pesticide companies. In such bactericides, pyrazole amide compounds have the largest number of varieties on the market, and many studies on the bactericides have been made so far.

WO 2012/065947 A1 describes 5-halogenopyrazole(thio)carboxamides and their use in methods for the control of phytopathogenic fungi.

CN1226244A and CN101056858A respectively disclose the formula of a pyrazole amide compound used as pesticide, and specifically disclose certain pyrazole amide compound containing a diphenyl ether structure in the Examples.

For example, CN1226244A discloses the following two pyrazole amide compounds containing a diphenyl ether group 12 and 21.

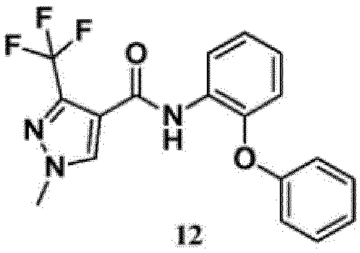
12

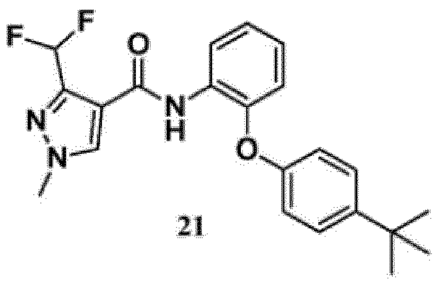
21

CN101056858A discloses a structure of pyrazole amide compound containing a diphenyl ether group substituted with an oxime.

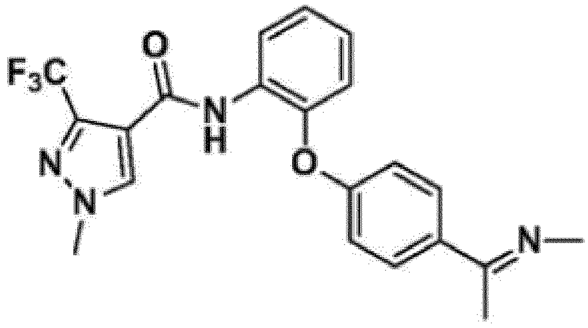

However, these pyrazole amide compounds containing a diphenyl ether group described in the prior art have poor effect on prevention and control the crop diseases and insect pests after the bioassay, especially poor effect on the rice sheath blight and cucumber powdery mildew which affect the food security of the country.

**Summary of the invention**

The object of the present invention is to provide a pyrazole amide compound containing a diphenyl ether group, which can be used to prevent and treat of rice sheath blight and/or cucumber powdery mildew effectively.

The inventors of the present invention unexpectedly found that two specific pyrazole amide compounds containing a diphenyl ether group (represented by formula I and formula II) which are not disclosed by the prior art have a very excellent bactericidal effect by screening studies. Compared to other pyrazole amide compounds containing a diphenyl ether group, the two specific compounds are able to prevent and treat rice sheath blight and / or cucumber powdery mildew effectively.

The present invention provides a pyrazole amide compound containing a diphenyl ether group represented by the following formula I and/or formula II,

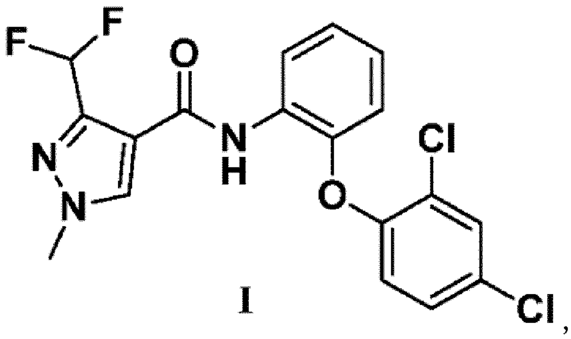

I ,

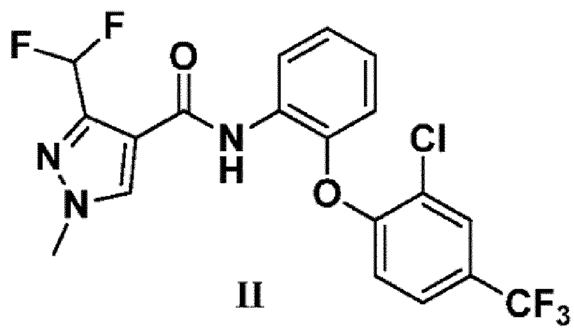

II

Furthermore, the present invention provides an application of the compound represented by formula I and/or formula II in the prevention and treatment of rice sheath blight.

Furthermore, the present invention provides an application of the compound represented by formula I and/or formula II in the prevention and treatment of cucumber powdery mildew.

Furthermore, the present invention provides a pesticide composition comprising the compound represented by formula I and/or formula II as an active ingredient.

Furthermore, the present invention provides an application of the compound represented by formula I and/or formula II in the preparation of pesticide in the prevention and treatment of rice sheath blight and cucumber powdery mildew at the same time.

Activity test results showed that the biological activity of the compounds represented by formula I and formula II in the present invention is far superior to other pyrazole amide compounds(including those two compounds implemented in Example No. 12 and 21 in CN1226244A), whether in enzyme level, or in vivo pot experiment. Through further test of pot experiment in vivo, the present compounds represented by formula I and formula II showed a very excellent effect in the prevention of rice sheath blight, better than the specific bactericide Thifluzamide used for the disease in the current market, meanwhile the results of field efficacy test also showed the same conclusion.

Other features and advantages of the invention will be described in detail in the following specific embodiments.

**Description of the invention in detail**

Hereinafter, embodiments of the present invention will be described in detail. It should be understood that the particular embodiments herein are only used for describing and illustrating the invention, and shall not be construed to limit the invention.

Pyrazole amide compound containing a diphenyl ether group represented by formula I in the present invention can be synthesized using the following synthetic route (1):

The synthesis of the compound I:

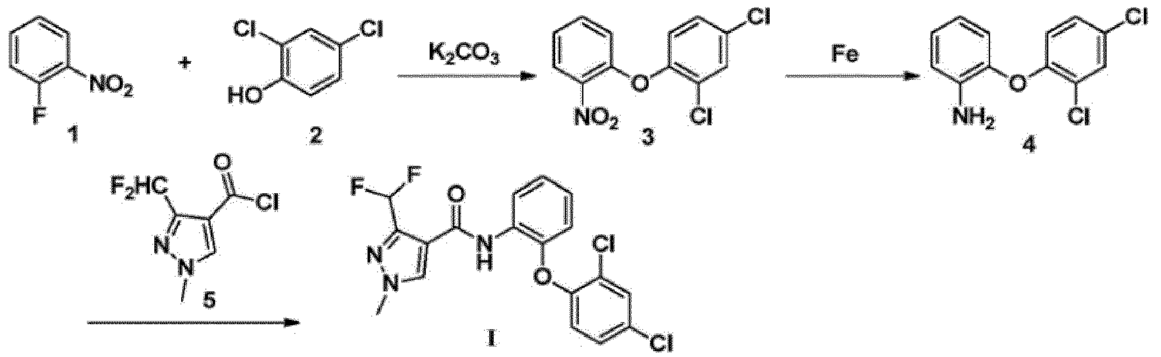


Synthetic route (1)

Pyrazole amide compound containing a diphenyl ether group represented by formula II in the present invention can be synthesized using the following synthetic route (2):

The synthesis of the compound II:

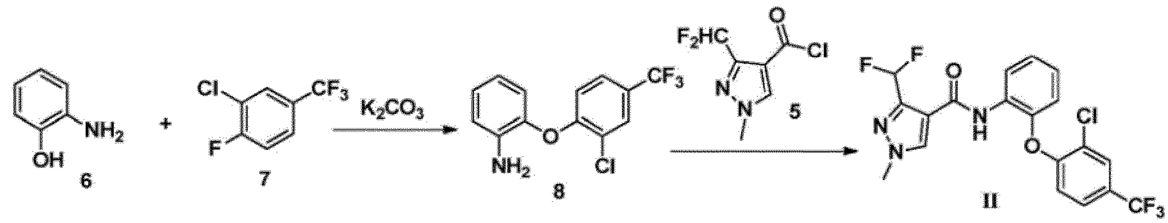


Synthetic route (2)

When the compounds provided in the present invention represented by formula I and/or formula II were used in the prevention and treatment of rice sheath blight and cucumber powdery mildew, the compounds represented by formula I and/or formula II as an active ingredient can be prepared into pesticide composition and applied to crops in an effective amount.

The expression of "an effective amount" may be suitably determined according to the specific circumstances of the crops to be applied. Under normal circumstances, the application amount of compounds shown in formula I and / or formula II was 1-20 grams per Mu.

The compounds of formula I and/or formula II can be made into a pesticide composition, the formulation of the pesticide composition may be present in the conventional formulation, for example, the compound of formula I and/or formula II was dissolved in a solvent, and then formulated into emulsifiable concentrates (EC). The solvent may be desirable as long as it can dissolve the pyrazole amide compound containing a diphenyl ether group represented by formula I and / or formula II, examples thereof may include, but are not limited to, N, N-dimethyl formamide (DMF). The emulsifiable concentrates may contain a surfactant conventionally used in the art, examples thereof include, but are not

limited to, 0.1% Tween-80.

The inventors of the present invention have found that the compounds represented by formula I and formula II have a very excellent effect in the prevention and treatment of rice sheath blight and cucumber powdery mildew. Therefore, it is preferable to apply the compounds represented by formula I and/or formula II in the preparation of pesticide for prevention and treatment of rice sheath blight and cucumber powdery mildew at the same time.

A detailed description of the invention will be described by means of examples. Unless otherwise specified, the various reagents in the following examples were purchased commercially.

Example 1

The synthesis of the pyrazole amide compounds containing a diphenyl ether group as represented by formula I and/or formula II.

With reference to the synthetic route (1) and synthetic route (2) as described above.

1. The synthesis of 2,4-dichloro-1-(2-nitrophenoxy)benzen.

2.82g (20mmol) 2-fluoro nitrobenzene, 4.89g (30mmol) 2,4-dichloro phenol, 4.15g (30mmol) $K_2CO_3$ and 50ml DMF were added into a 100ml single neck flask, and heated under reflux for 5h, then DMF was removed under reduced pressure, the reaction mixture was washed three times with 10% NaOH aqueous solution, extracted with ethyl acetate, dried with anhydrous $Na_2SO_4$, and evaporated to give 5.50g 2,4-dichloro-1-(2-nitrophenoxy)benzene with yield of 97%.

2. The synthesis of 2-(2,4-dichlorophenoxy)aniline

5.50g (19.36mmol) 2,4-dichloro-1-(2-nitrophenoxy) benzene was dissolved in 100ml of ethanol, 1.04g (19.36mmol) of ammonium chloride and 10ml of water were added thereto, and heated under reflux, then 3.24g (58.08mmol) of iron powder was added thereto in three lots, after 6h reaction, the reaction system was filtered by suction with silica gel, evaporated to remove ethanol, then diluted with 100ml of ethyl acetate, and washed with saturated brine 60ml $\times$ 3 times, dried and evaporated to give 4.50g 2- (2,4-dichlorophenoxy) aniline with yield of 91%.

3. The synthesis of 2-(2-chloro-4-trifluoromethyl-phenoxy) aniline

5.00g (45.82mmol) 2-aminophenol, 9.10g (45.82mmol) 3-chloro-4-fluoro benzotrifluoride, 9.50g (68.73mmol) $K_2CO_3$ and 100ml DMF were added into a single neck flask of 250ml, and heated under reflux for 5h, then DMF was removed under reduced pressure, the reaction mixture was washed with 10% NaOH aqueous solution three times, extracted with ethyl acetate, dried with anhydrous $Na_2SO_4$, and evaporated to give 9.53 g 2-(2-chloro-4-trifluoromethyl-phenoxy) aniline with yield of 72%.

4. The synthesis of compound I

0.50g (1.97mmol) 2-(2,4-dichlorophenoxy) aniline was dissolved in 20ml of dichloromethane, and 0.30g (2.95mmol) of triethylamine was added, then 3-difluoromethyl-1-methyl-1H-pyrazole-4-formyl chloride 0.46g (2.36mmol) was dropped slowly under ice bath. The reaction system was monitored by TLC, diluted with 30ml of dichloromethane after completion of the reaction, washed with saturated brine 50ml $\times$ 3 times, dried with anhydrous sodium sulfate, and purified by column chromatography to give 0.70g compound I with yield of 86%.

5. The synthesis of compound II

0.57g (1.97mmol)2-(2,4-dichlorophenoxy)aniline was dissolved in 20ml of dichloromethane, 0.30g (2.95mmol) of triethylamine was added, then 3-difluoromethyl-1-methyl-1H-pyrazole-4-formyl chloride 0.46g (2.36mmol) was dropped slowly under ice bath. The reaction system was monitored by TLC, diluted with 30ml of dichloromethane after completion of the reaction, washed with saturated brine 50ml $\times$ 3 times, dried with anhydrous sodium sulfate, and purified by column chromatography to give 0.75g compound I with yield of 85%.

Related NMR spectral data of compounds I and II are as follows:

Compound I: $^1$HNMR (600MHz,$CDCl_3$) $\delta$ 8.67(s,1H), 8.55(d,J=8.4Hz,1H), 7.99(s,1H), 7.48(t,J=8.4Hz,1H), 7.21(m,2H), 7.04(s,1H), 6.92(t,J=48.6Hz,1H), 6.74(d,J=13.8Hz,1H), 3.95(s,3H). MS:m/z=411.29(M+). m.p.:102.5-103.6°C.

Compound II: $^1$HNMR (600MHz,$CDCl_3$) $\delta$ 8.62(s,1H), 8.58(d,J=8.4Hz,1H), 7.99(s,1H), 7.75(s,1H),

7.46(d,J=9Hz,1H), 7.24(d,J=7.8Hz,1H), 7.11(t,J=7.8Hz,1H), 6.99(d,J=9Hz,1H), 6.87(t,J=53.4Hz,1H), 6.86(s,1H),3.92(s,3H). MS:m/z=445.15(M+). m.p.:114.3-115.4°C.

Preparation example 1

The comparative compounds represented by formula III with a similar structure of the pyrazole amide compound represented by formula I and/or formula II can be synthesized using a similar synthetic route as that in Example 1, numbers of the synthesized comparative compounds were listed in table 1.

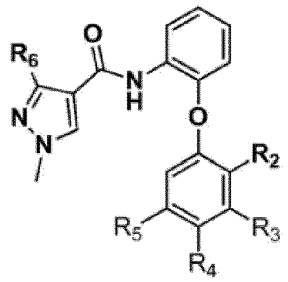

III

Table 1

| Number | $R_6$ | $R_2$ | $R_3$ | $R_4$ | $R_5$ |
|---|---|---|---|---|---|
| III-1 | $CF_3$ | H | Cl | H | H |
| III-2 | $CF_3$ | Cl | H | Cl | H |
| III-3 | $CF_3$ | H | H | F | H |
| III-4 | $CF_3$ | H | OMe | OMe | OMe |
| III-5 | $CHF_2$ | H | OMe | OMe | OMe |
| III-6 | $CF_3$ | H | H | CN | H |
| III-7 | $CHF_2$ | H | H | CN | H |
| III-8 | $CF_3$ | Cl | H | H | H |
| III-9 | $CF_3$ | H | H | Cl | H |
| III-10 | $CHF_2$ | H | H | Cl | H |
| III-11 | $CF_3$ | H | H | $CF_3$ | H |
| III-12 | $CHF_2$ | H | H | $CF_3$ | H |
| III-13 | $CF_3$ | $CH_3$ | $CH_3$ | H | H |
| III-14 | $CHF_2$ | $CH_3$ | $CH_3$ | H | H |
| III-15 | $CF_3$ | H | $CH_3$ | H | $CH_3$ |
| III-16 | $CHF_2$ | H | $CH_3$ | H | $CH_3$ |

HNMR spectral data of the above compounds are as follows:

III-1: $^1$HNMR (600MHz,$CDCl_3$) $\delta$ 8.55(d,J=7.8Hz,1H), 8.38(s,1H), 7.98(s,1H), 7.20(t,J=7.8Hz,1H), 7.11(m,3H), 7.03(t,J=4.2Hz,2H), 6.93(d,J=7.8Hz,1H), 3.98(s,3H). MS:m/z=395.27(M+). m.p.:74.5-75.9°C.
III-2: $^1$HNMR (600MHz,$CDCl_3$) $\delta$ 8.53(d,J=1.8Hz,1H), 8.51(s,1H),8.01(s,1H), 7.49(t,J=1.8Hz,1H), 7.24-7.14(m,2H), 7.06(d,J=1.8Hz,1H), 7.04(m,1H), 6.74(m,1H), 3.98(s,3H). MS:m/z=429.26(M+). m.p.:93.2-94.0°C.
III-3: $^1$HNMR (600MHz,$CDCl_3$) $\delta$ 8.54(d,J=7.8Hz,1H), 8.47(s,1H), 7.99(s,1H), 7.16(t,J=7.8Hz,1H), 7.07(m,3H),7.00(d,J=4.2Hz,2H), 6.84(s,1H), 6.74(m,J=7.8Hz,1H), 3.98(s,3H). MS:m/z=379.33(M+). m.p.:127.5-128.0°C.
III-4: $^1$HNMR (600MHz,$CDCl_3$) $\delta$ 8.51(d,J=12Hz,1H), 8.44(s,1H), 7.98(s,1H), 7.13(d,J=12Hz,1H), 7.08(m,1H),

6.91(d,J=12Hz,1H), 6.29(s,2H), 3.98(s,3H), 3.83(s,3H), 3.79(s,6H). MS:m/z=451.15(M+). m.p.:155.5-156.4°C.

III-5: $^1$HNMR (600MHz,$CDCl_3$) δ 8.68(s,1H), 8.51(d,J=12Hz,1H), 7.96(s,1H), 7.14(t,J=12Hz,1H), 7.06(m,1H), 7.03(t,J=81Hz,1H), 6.91(t,J=1.8Hz,1H), 6.29(s,2H), 3.92(s,3H), 3.84(s,3H), 3.79(s,6H). MS:m/z=433.17(M+). m.p.:136.7-137.5°C.

III-6: $^1$HNMR (600MHz,$CDCl_3$) δ 8.73(d,J=7.8Hz,1H), 8.49(s,1H), 7.98(s,1H), 7.43(d,J=7.8Hz,1H), 7.19(d,J=8.4Hz,2H), 7.08(m,2H), 6.87(d,J=7.8Hz,2H), 3.97(s,3H). MS:m/z=387.43(M+). m.p.:156.5-157.3°C.

III-7: $^1$HNMR (600MHz,$CDCl_3$) δ 8.69(s,1H), 8.58(d,J=7.8Hz,1H), 7.97(s,1H), 7.29(t,J=8.4Hz,1H), 7.03(m,3H), 6.95(m,3H), 6.97(t,J=56.4Hz,1H), 3.97(s,3H). MS:m/z=368.37(M+). m.p.:137.9-139.2°C.

III-8: $^1$HNMR (600MHz,$CDCl_3$) δ 8.54(d,J=7.8Hz,1H), 8.47(s,1H), 7.98(s,1H), 7.50(t,J=7.8Hz,1H), 7.16(m,3H), 7.02(t,J=4.2Hz,2H), 6.74(d,J=7.8Hz,1H), 3.98(s,3H).MS: m/z=395.48(M+). m.p.:80.5-81.9°C.

III-9: $^1$HNMR (600MHz,$CDCl_3$) δ 8.54(d,J=7.8Hz,1H), 8.40(s,1H), 7.98(s,1H), 7.31(d,J=7.8Hz,1H), 7.17(d,J=8.4Hz,2H), 7.08(t,J=8.4Hz,1H), 6.97(d,J=7.2Hz,2H), 6.90(d,J=7.8Hz,2H), 3.97(s,3H). MS:m/z=396.43(M+). m.p.:88.2-88.7°C.

III-10: $^1$HNMR(600MHz,$CDCl_3$) δ 8.66(s,1H), 8.57(d,J=8.4Hz,1H), 7.95(s,1H), 7.46(t,J=8.4Hz,1H), 7.21(m,3H), 6.93(m,3H), 6.88(t,J=53.4Hz,1H), 3.99(s,3H). MS:m/z=377.56(M+). m.p.:100.7-101.9°C.

III-11: $^1$HNMR (600MHz,$CDCl_3$) δ 8.56(d,J=7.2Hz,1H), 8.33(s,1H),7.97(s,1H), 7.61(d,J=8.4Hz,2H), 7.25(t,J=7.8Hz,1H), 7.13(d,J=7.8Hz,1H), 7.10(d,J=8.4Hz,2H), 6.98(d,J=7.8Hz,1H), 3.97(s,3H). MS:m/z=429.15(M+). m.p.:105.4-106.2°C.

111-12: $^1$HNMR (600MHz,$CDCl_3$) δ 8.65(s,1H), 8.58(d,J=8.4Hz,1H), 7.97(s,1H), 7.59(d,J=8.4Hz,2H), 7.25(t,J=7.2Hz,1H), 7.12(d,J=7.8Hz,1H), 7.09(d,J=7.8Hz,1H), 6.99(d,J=14.4Hz,1H), 6.85(t,J=53.4Hz,1H), 3.92(s,3H). MS:m/z=411.17(M+). m.p.:108.4-109.8°C.

111-13: $^1$HNMR (600MHz,$CDCl_3$) δ 8.60(s,1H), 8.54(d,J=12Hz,1H), 8.00(s,1H), 7.10(m,4H), 6.95(d,J=12Hz,1H), 6.62(d,J=12Hz,1H), 3.97(s,3H), 2.34(s,3H), 2.15(s,3H). MS:m/z=389.14(M+). m.p.:120.7-122.1°C.

111-14: $^1$HNMR (600MHz,$CDCl_3$) δ 8.83(s,1H), 8.54(d,J=12Hz,1H), 7.96(s,1H), 7.09(m,4H), 7.06(m,4H), 7.01(t,J=81Hz,1H), 6.79(d,J=12Hz,1H), 6.62(d,J=12.6Hz,1H), 3.91(s,3H), 2.32(s,3H), 2.15(s,3H). MS:m/z=371.16(M+). m.p.:143.6-145.1°C.

111-15: $^1$HNMR (600MHz,$CDCl_3$) δ 8.54(s,1H), 8.52(d,J=12Hz,1H), 7.92(s,1H), 7.13(m,1H), 7.04(m,1H), 6.89(d,J=12Hz,1H), 6.78(s,1H), 6.65(s,2H), 3.97(s,3H),2.29(s,6H). MS:m/z=389.14(M+). m.p.:103.2-104.3°C.

III-16: $^1$HNMR (600MHz,$CDCl_3$) δ 8.66(s,1H), 8.54(d,J=8.4Hz,1H), 7.90(s,1H), 7.13(d,J=7.2Hz,1H), 7.03(m,1H), 6.97(t,J=54Hz,1H), 6.89(s,1H),6.77(s,1H), 6.77(s,2H), 3.94(s,3H), 2.29(s,6H). MS:m/z=371.16(M+). m.p.:93.8-94.7°C.

Preparation example 2

Compounds 12 and 21 represented by the following formulas were prepared according to the method of embodiments 12 and 21 disclosed in CN1226244A.

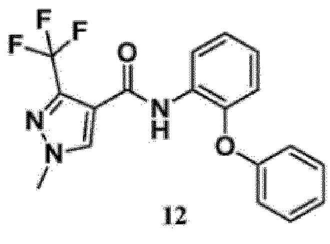

12

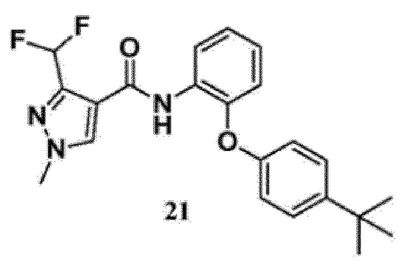

21

Test Example 1

This test was used to determine the inhibitory activity on the succinate dehydrogenase of the compounds prepared in Example 1, Preparation Examples 1 and 2.

The enzyme used in the test example was succinate dehydrogenase, which was isolated from pig hearts.

Test Method: total volume was 1.8ml, the system comprised 100mM $Na_2HPO_4$-$NaH_2PO_4$ buffer (pH7.4), 0.3mM EDTA, 20mM sodium succinate, 53μM DCIP (2,6-dichloro indophenol sodium), 2nM succinate dehydrogenase. In water bath with 23°C of constant temperature and 600rpm magnetic stirring, the reduction of the light absorption of substrate DCIP was monitored at a wavelength of 600nm, the experimental points within the linear range were collected, that is, the experimental points in which the consumption of substrate was controlled not exceeding 5% were collected. The molar extinction coefficient of DCIP was $21mM^{-1}cm^{-1}$. The reduction amount of DCIP during the reaction time was

calculated and linear slope was fitted, and then the initial velocity of the reaction was obtained by deducting the baseline slope.

The data of inhibitory activity of the compounds on the succinate dehydrogenase were shown in table 2.

Table 2

| Number | $IC_{50}$ (μM) |
|---|---|
| Comparative compound III-1 | 36.89±1.47 |
| Comparative compound III-2 | 2.70±1.37 |
| Comparative compound III-3 | - |
| Comparative compound III-4 | - |
| Comparative compound III-5 | - |
| Comparative compound III-6 | - |
| Comparative compound III-7 | - |
| Comparative compound III-8 | 1.98±1.39 |
| Comparative compound III-9 | 4.90±1.28 |
| Comparative compound III-10 | 2.02±1.15 |
| Comparative compound III-11 | 3.17±1.12 |
| Comparative compound III-12 | 1.62±1.35 |
| Comparative compound III-13 | 9.86±1.27 |
| Comparative compound III-14 | 3.22±1.33 |
| Comparative compound III-15 | 5.12±1.04 |
| Comparative compound III-16 | 2.03±1.22 |
| I | 0.401±0.0103 |
| II | 0.0484±0.00171 |
| Comparative compound 12 (CN1226244A) | 19.8±1.07 |
| Comparative compound 21 (CN1226244A) | 1.18±0.0137 |
| Thifluzamide (Produced by Hubei Jianhe Chemical Co. Ltd) | 0.200±0.0121 |
| Note: "-" indicates that the value of $IC_{50}$ is greater than 100μM. | |

The results in table 2 show that pyrazole amide compounds containing a diphenyl ether group as represented by formula I and formula II have inhibitory effect on the activity of the enzyme, and the value of $IC_{50}$ thereof is significantly lower than that of the control compounds, which means that the inhibitory effect on the activity of the enzyme thereof is high.

The data of inhibitory activity on the succinate dehydrogenase of compounds I, II and comparative compounds 12 and 21 at different concentrations were shown in table 3.

Table 3

| Number | concentration | inhibition ratio(%) |
|---|---|---|
| I | 50 μM | 73.01 |
| | 10 μM | 74.44 |
| | 1 μM | 66.87 |
| | 0.5 μM | 52.28 |
| | 0.3 M | 42.13 |
| | 0.2 μM | 37.42 |
| | 0.1 μM | 26.99 |
| | 0.05 μM | 21.65 |
| | 0.03 μM | 18.34 |
| II | 50 μM | 100.0 |
| | 10 μM | 97.86 |
| | 1 μM | 91.44 |
| | 0.5 μM | 83.81 |
| | 0.2 μM | 75.32 |
| | 0.1 μM | 60.74 |
| | 0.05 μM | 47.09 |
| | 0.03 μM | 31.84 |
| | 0.02 μM | 20.80 |
| | 0.01 μM | 11.71 |
| comparative compound 12 | 50 μM | 61.78 |
| | 30 μM | 50.33 |
| | 20 μM | 38.05 |
| | 10 μM | 22.31 |
| | 5 μM | 10.02 |
| | 1 μM | 0 |
| comparative compound 21 | 50 μM | 80.77 |
| | 1.0 μM | 79.36 |
| | 3 μM | 60.73 |
| | 1 μM | 46.92 |
| | 0.5 μM | 34.60 |
| | 0.3 μM | 27.39 |
| | 0.2 μM | 24.72 |
| | 0.1 μM | 0 |

(continued)

| Number | concentration | inhibition ratio(%) |
|---|---|---|
| Thifluzamide | 50 μM | 96.73 |
| | 10 μM | 94.55 |
| | 1 μM | 77.93 |
| | 0.5 μM | 71.72 |
| | 0.3 μM | 59.52 |
| | 0.2 μM | 47.63 |
| | 0.1 μM | 29.70 |
| | 0.05 μM | 9.21 |
| | 0.03 μM | 0 |

As shown in table 3, compared with the comparative compounds 12, 21 and the control bactericide Thifluzamide, the inhibitory activity on the succinate dehydrogenase of compounds I, II are higher than or commensurate with the control bactericide at the same concentration, however, when the concentration was decreased, the inhibitory activity on the succinate dehydrogenase of the comparative compounds 12, 21 and the control bactericide Thifluzamide were basically loss or decreased significantly, while the compounds I and II showed good inhibitory activity even at low concentration.

Test Example 2

This test is used to determine the bactericidal activity of the compounds prepared in Example 1, Preparation Examples 1 and 2.

The targets used in the test example are rice sheath blight and cucumber powdery mildew.

Test Method: According to "Standard Practice for determination of pesticide biological activity (SOP)", pyrazole amide compound containing a diphenyl ether group was dispensed to 5% emulsifiable concentrates (hereinafter referred to as 5% EC), the bactericidal activity of these compounds on the two test targets in the dose of 200mg/L was evaluated using the following test method (pot in vivo assay). The screening results on the two test targets of the compounds prepared in Example 1, Preparation Examples 1 and 2 were shown in Table 4.

Cucumber powdery mildew (Sphaerotheca uliginea)

A cucumber seedling with uniform growth in true leaf stage was chosen, and dried in shade for 24h after spray treatment. Fresh cucumber powdery mildew spores on the cucumber leaves were collected and washed, and filtered through a double gauze to prepare a spore suspension with concentration of about 100,000 / ml, then spray inoculation was applied. The test materials inoculated were moved into artificial climate with relative humidity of 60-70% and temperature of 21-23 °C (indoor lighting), after about 10 days, investigating and grading were carried out according to the incidence of the blank control, and the control effect was calculated according to the disease index.

Rice sheath blight (Rhizoctonia solani)

Two potted rice seedlings with uniform growth in true leaf stage were chosen, and dried by air after spray treatment. The side of bacteria cake with mycelium was closely attached on the leaves, after cultured at 22-26 °C under dark light in a moisture preserving manner for 24h, then cultured with natural light for about 4 days. The diameter of disease spot of each inoculating point was measured by using a caliper after the control material was fully attacked by disease, and the control effect was calculated.

Investigation method and grading standard adopted "Guidelines for the field efficacy trials of pesticides", and the control effect was calculated with the disease index.

The disease index = Σ (number of diseased leaves at each grade × relative grade value) × 100 / (total number of leaves × 9);

Control effect (%) = (disease index of control - disease index of treated) × 100 / disease index of control.

Table 4

| Number | concentration (mg/L) | Efficacy(%) | |
|---|---|---|---|
| | | rice sheath blight | cucumber powery mildew |
| comparative compound III-1 | 200 | 68.61 | 22.22 |
| comparative compound III-2 | 200 | 0 | 72.22 |
| comparative compound III-3 | 200 | 56.95 | 0 |
| comparative compound III-4 | 200 | 0 | 0 |
| comparative compound III-5 | 200 | 0 | 0 |
| comparative compound III-6 | 200 | 30.18 | 0 |
| comparative compound III-7 | 200 | 45.02 | 0 |
| comparative compound III-8 | 200 | 81.94 | 0 |
| comparative compound III-9 | 200 | 66.29 | 0 |
| comparative compound III-10 | 200 | 68.30 | 0 |
| comparative compound III-11 | 200 | 56.26 | 0 |
| comparative compound III-12 | 200 | 72.31 | 0 |
| comparative compound III-13 | 200 | 63.23 | 0 |
| comparative compound III-14 | 200 | 65.92 | 0 |
| comparative compound III-15 | 200 | 55.23 | 0 |
| comparative compound III-16 | 200 | 50.02 | 0 |
| I | 200 | 100 | 100 |
| II | 200 | 100 | 100 |
| **comparative compound 12** | 200 | 30.87 | 0 |
| **comparative compound 21** | 200 | 22.31 | 0 |

The results of secondary screening on rice sheath blight and cucumber powdery mildew of compounds I, II and the control bactericide Thifluzamide were shown in table 5.

Table 5

| Number | concentration (mg/L) | efficacy on rice sheath bilght (%) | efficacy on cucumber powery mildew(%) |
|---|---|---|---|
| I | 100 | 100 | 0 |
| | 50 | 98 | - |
| | 25 | 95 | - |
| | 12.5 | 81.72 | - |
| | 6.25 | 68.77 | - |
| | 3.125 | 65.37 | - |
| | 1.5 | 45.38 | - |
| | 1 | 20.74 | - |
| II | 100 | 100 | 100 |
| | 50 | 100 | 100 |
| | 25 | 98.66 | 94.44 |
| | 12.5 | 94.62 | 66.67 |
| | 6.25 | 70.43 | 0 |
| | 3.125 | 68.68 | - |
| | 1.5 | 55.34 | - |
| | 0.75 | 43.75 | - |
| Thifluzamide | 100 | 100 | 100 |
| | 50 | 100 | 62.96 |
| | 25 | 81.85 | 22.22 |
| | 12.5 | 61.36 | 0 |
| | 6.25 | 54.30 | - |
| | 3.125 | 13.38 | - |
| | 1.5 | 0 | - |
| | 0.75 | - | - |
| Note: "-" indicates the loss of bactericidal activity. | | | |

As shown in table 4, at concentration of 200 mg / L, the control effect of compounds I and II on rice sheath blight reached 100%, meanwhile the control effect on cucumber powdery mildew reached 100%. As shown in table 5, compared with the special effect bactericide Thifluzamide for rice sheath blight, the control effect of compounds I and II on rice sheath blight is better than the control bactericide Thifluzamide at the same concentration, especially at low concentrations. Meanwhile, the control effect of compound II on cucumber powdery mildew also showed good results, better than the commercial control bactericide Thifluzamide.

Test Example 3

According to " Guidelines for the field efficacy trials of pesticides (a) - Insecticides against rice sheath blight" established by Institute for the Control of Agrochemicals, Ministry of Agriculture, the field efficacy trials were conducted with 5% compound I EC and 5% compound II EC in the prevention and treatment of rice sheath blight.

1. Selection of the test subject, crops and varieties

Test subject: sheath blight Thanatephorus cucumeris (Frank) Donk .

Test crops: rice; Cultivars: South japonica 46.

The test plots were located in Fanpu village, Sunduan town, Shaoxing County, Zhejiang Province, with good water and fertilizer conditions, and the cultivation and management conditions were conformity with the test requirements.

2. Test agents

5% compound I EC, 5% compound II EC, 2.5% Thifluzamide EC, and a water control treatment. The dosage and treatment number in the design for trials of pesticides were shown in table 6.

Table 6

| Number | agents | Dosage per MU (ml) |
|---|---|---|
| 1 | 5% compound I EC | 100 |
| 2 | 5% compound II EC | 100 |
| 3 | 2.5% Thifluzamide EC | 200 |
| 4 | CK | - |

3. The arrangement of the field plot

The plots were distributed randomly according to block, the field was north-south direction, specific plot distribution was shown in table 7:

Table 7

| | | | |
|---|---|---|---|
| 1 | 8 | 6 | 4 |
| 2 | 9 | 7 | 5 |
| 3 | 1 | 8 | 6 |
| 4 | 2 | 9 | 7 |
| 5 | 3 | 1 | 8 |
| 6 | 4 | 2 | 9 |
| 7 | 5 | 3 | 1 |
| 8 | 6 | 4 | 2 |
| 9 | 7 | 5 | 3 |

Plot area: 30 square meters.
Number of repetation: 4 times.

4. The method of administration

Stem and leaf spraying treatment was conducted, the test agents were firstly administered from low concentration to high concentration sequentially, then the control agents, the sprayer was cleaned when changing agents.

A hand-operated sprayer 3Wbs-16, produced by Taizhou Guangfeng Plastic Co., Ltd., Zhejiang Province, was used as the spraying equipment, with working pressure 0.2mpa.

The total times of administration in this test were 2, on August 4, 2013 (at incipient stage of the sheath blight) and August 11, 2013 respectively.

The application dosage was 675L / hectare.

During the growth period of rice, no prevention and treatment for other diseases had been made, and the control on diseases and insect pests was the same as conventional rice.

5. Methods of investigation, recording and measurement

5.1. Meteorological and soil data

On the day of first administration, cloudy, southerly winds 2-3 grade, the temperature 29.0 °C ~ 37.8 °C, relative humidity 58%; on the day of second administration, sunny to cloudy, southerly winds 1-2 grade , the temperature 30.1 °C ~ 40.6 °C, relative humidity 45%. See the table of meteorological data for detail.

The testing field was flat, the soil was yellow purple clay, with organic matter content of 2.4%, pH value of 6.7.

5.2. Methods, time and frequency of investigation

The total investigation times in this test was 1, the investigation on the testing results was conducted on August 25, that is, 14 days after the second administration.

According to the provisions of " Guidelines for the field efficacy trials of pesticides (a) - Insecticides against rice sheath blight", samples were taken for each test plot adopting diagonal five-point sampling method, and 20 neighboring strains were investigated for each point, 100 strains in total, the morbidity number and disease grade were recorded. The grade standard of diseases was as follows:

Grade 0: whole plant had no disease;
Grade 1: each leaf and sheath of the fourth leaf and below the fourth leaf were attacked (taking the first flag leaf as the first leaf);
Grade 3: each leaf and sheath of the third leaf and below the third leaf were attacked;
Grade 5: each leaf and sheath of the second leaf and below the second leaf were attacked;
Grade 7: each leaf and sheath of the flag leaf and below the flag leaf were attacked;
Grade 9: whole plant was attacked, and early death.

Efficacy calculation method: According to the investigation results, the disease index and control effect were calculated in accordance with the following formula (1), (2). The test data were statistically analyzed using Duncan's multiple range test (DMRT).

$$\text{Disease index} = \frac{\sum[\text{Number of diseased leaves at each grade} \times \text{relative grade value}]}{\text{total investigated leaves} \times 9} \times 100 \quad \text{...........(1)}$$

$$\text{Control effect (\%)} = \left(1 - \frac{\text{disease index after administration in treated region}}{\text{disease index after administration in blank control region}}\right) \times 100 \quad \text{............(2)}$$

No benificial or adverse effects of the experimental agents on crops were observed, and no effects on other diseases and insect pests were observed during the test.

6. Results of the test

The results of the field efficacy trials of 5% compound I EC and 5% compound II EC were showed in table 8.

Table 8

| Number (dosage) | Disease Index(%) | Efficacy(%) |
|---|---|---|
| 5% compound I 100ml/Mu | 2.96 | 77.16 |
| 5% compound II EC 100ml/Mu | 2.78 | 79.94 |
| 2.5% Thifluzamide EC 200ml/Mu | 2.93 | 74.51 |
| CK | 13.31 | / |

The results of the field efficacy trials of 5% compound I EC and 5% compound II EC show that, the control effect of the compounds of the present invention on the rice sheath blight is better than the commercially available fungicide Thifluzamide at the same dose.

It can be seen from the results of test examples 1 to 3, the control effect of the compounds I and II of the present invention on the rice sheath blight is not only much better than other similar pyrazole amide compounds containing a diphenyl ether group (including compounds 12 and 21 disclosed in the art CN1226244A), but also superior to the commercially available specific bactericide for rice sheath blight---Thifluzamide at the same dose. Even when used at low concentrations, the compounds I and II compounds of the invention also show good results in the control of rice sheath blight. Accordingly, the compounds I and II of the present invention obtain unexpected technical effect.

Preferred embodiments of the present invention have been described in detail above.

It also should be noted that the various specific technical features of the above described embodiments may be combined in any suitable manner in the case of non-contradiction. In order to avoid unnecessary duplication, no further specific description will be made about each possible combinations of the present invention.

In addition, any combination among various embodiments of the present invention may also be carried out, and should be regarded as the content of the present invention, as long as it is not in contrary to the idea of the present invention.

## Claims

1. A pyrazole amide compound containing a diphenyl ether group represented by the following formula I or formula II,

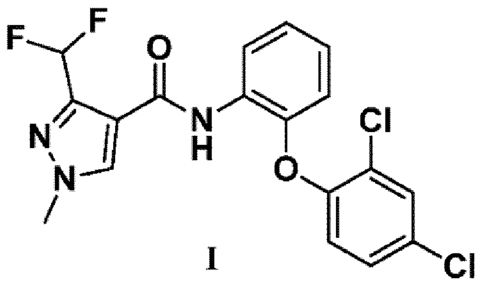

I ,

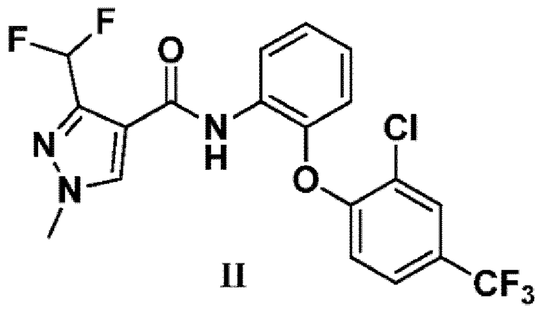

II .

2. An application of the compound according to claim 1 in the prevention and treatment of rice sheath blight.

3. An application of the compound according to claim 1 in the prevention and treatment of cucumber powdery mildew.

4. A pesticide composition comprising the compound of claim 1 as an active ingredient.

5. An application of the compound according to claim 1 in the preparation of pesticide for prevention and treatment of rice sheath blight and cucumber powdery mildew at the same time.

## Patentansprüche

1. Pyrazolamidverbindung, die eine Diphenylethergruppe enthält, dargestellt durch die folgende Formel I oder Formel II:

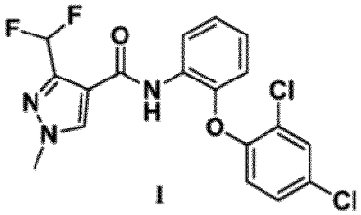

I

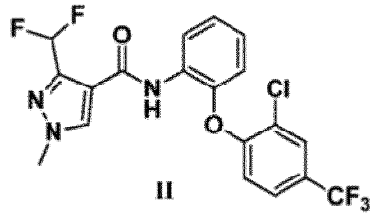

II .

2. Verwendung der Verbindung nach Anspruch 1 zur Vorbeugung und Behandlung von Blattscheidenbrand bei Reis.

3. Verwendung der Verbindung nach Anspruch 1 zur Vorbeugung und Behandlung von Echtem Mehltau bei Gurken.

4. Pestizidzusammensetzung, umfassend die Verbindung nach Anspruch 1 als Wirkstoff.

5. Verwendung der Verbindung nach Anspruch 1 bei der Herstellung von Pestiziden zur gleichzeitigen Vorbeugung und Behandlung von Blattscheidenbrand bei Reis und Echtem Mehltau bei Gurken.

## Revendications

1. Composé de pyrazole-amide contenant un groupe éther diphénylique, le composé étant représenté par les formules suivantes I ou II:

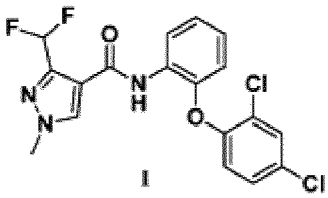

I

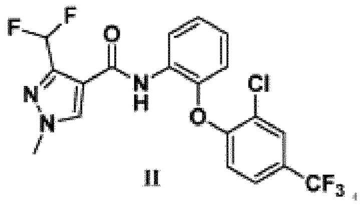

II

2. Application du composé selon la revendication 1 dans la prévention et le contrôle du flétrissement de la gaine du riz.

3. Application du composé selon la revendication 1 dans la prévention et le contrôle de l'oïdium du concombre.

4. Combinaison des pesticides comprenant le composé selon la revendication 1 comme ingrédient actif.

5. Application du composé selon la revendication 1 dans la préparation de pesticides pour la prévention et le contrôle du flétrissement de la gaine du riz et de l'oïdium du concombre en même temps.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2012065947 A1
- CN 1226244 A
- CN 101056858 A